(19) Europäisches Patentamt
European Patent Office
Office européen des brevets

(11) **EP 3 030 543 B1**

(12) **FASCICULE DE BREVET EUROPÉEN**

(45) Date de publication et mention
de la délivrance du brevet:
**25.07.2018 Bulletin 2018/30**

(51) Int Cl.:
***C07C 51/363*** *(2006.01)*     ***C07C 53/19*** *(2006.01)*
***C07C 227/08*** *(2006.01)*     ***C07C 229/08*** *(2006.01)*

(21) Numéro de dépôt: **14790193.8**

(86) Numéro de dépôt international:
**PCT/FR2014/052062**

(22) Date de dépôt: **07.08.2014**

(87) Numéro de publication internationale:
**WO 2015/019028 (12.02.2015 Gazette 2015/06)**

(54) **PROCÉDÉ D'HYDROBROMURATION**

HYDROBROMIERUNGSVERFAHREN

HYDROBROMINATION METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.08.2013 FR 1357928**

(43) Date de publication de la demande:
**15.06.2016 Bulletin 2016/24**

(73) Titulaire: **Arkema France**
**92700 Colombes (FR)**

(72) Inventeurs:
  • **BERTHE, Bernard**
    **F-64140 Lons (FR)**
  • **PEES, Bernard**
    **F-13190 Allauch (FR)**
  • **ANNOOT, Philippe**
    **F-27410 Saint Aubin le Guichard (FR)**
  • **DUBOIS, Jean-Luc**
    **F-69390 Millery (FR)**
  • **ZOVI, Ornella**
    **F-27230 Le Theil Nolent (FR)**

(74) Mandataire: **Hérard, Elise et al**
    **ARKEMA France**
    **Département Propriété Industrielle**
    **420, rue d'Estienne d'Orves**
    **92705 Colombes Cedex (FR)**

(56) Documents cités:
    **WO-A2-2007/121228     CN-A- 102 531 879**
    **FR-A- 951 932**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne un procédé continu de fabrication d'un composé de formule R1-CHBr-CH$_2$-R2 (de préférence Br-(CH$_2$)$_2$-R2, par exemple Br-(CH$_2$)$_2$-R'-COOH) par hydrobromuration (ou hydrobromation) à partir d'un composé I de formule :
R1-CH=CH-R2 (de préférence CH$_2$=CH-R, par exemple CH$_2$=CH-R'-COOH), dans laquelle R1, R2 identiques ou différents sont chacun indépendamment H ou un radical alkyle comportant de 1 à 30 atomes de carbone, de préférence de 6 à 15 atomes de carbone, saturé ou insaturé (alcène, alcyne), linéaire ou ramifié, fonctionnalisé (notamment par au moins une fonction acide, ester, nitrile, alcool et/ou aldéhyde) ou non fonctionnalisé.

La présente invention porte tout particulièrement sur un nouveau procédé de synthèse d'acide α,ω-bromo-aicanoïque (ci-après ω-bromoacide) susceptible d'être utilisé dans l'industrie des polymères, notamment des polyamides, ledit procédé comprenant une étape d'hydrobromuration radicalaire d'un acide ω-insaturé en présence de HBr, ce dernier arrivant dans la réaction sous forme de liquide (c'est-à-dire soit liquéfié sous sa pression de vapeur saturante soit en solution dans un solvant ou mélange de solvant).

Parmi les ω-bromoacides, l'acide 11-bromoundécanoïque (aussi appelé l'acide 11 Bromo), trouve de nombreuses applications dans l'industrie, notamment grâce à la présence de ses deux fonctions réactives, bromure et acide, qui sont susceptibles de réagir. L'acide 11 Bromo est le produit de départ d'un grand nombre de réactions chimiques industrielles, en particulier pour la fabrication de l'acide 11-aminoundécanoïque, utilisé comme monomère de synthèse du polyamide 11.

Art antérieur

Actuellement, deux procédés d'hydrobromuration sont décrits dans la littérature, utilisant un dispositif de colonne ou tour pour réaliser la réaction d'hydrobromuration. Le procédé décrit dans le document de brevet FR951932, utilise une dissolution d'acide undécylénique dans un mélange de benzène et de toluène, le mélange au préalable refroidi à -10°C coule dans une tour remplie d'anneaux de Raschig. Un mélange gazeux constitué d'acide bromhydrique et d'air est introduit dans la tour à contre-courant. La réaction se passe dans un espace réduit de la colonne. Dans cette zone, la température monte à 30°C. Le rendement en acide 11-bromoundécanoïque est dans ces conditions compris dans la gamme de 95 à 97%.
Le document de brevet CN1100030C décrit un procédé d'hydrobromuration en 2 étapes, absorption et réaction, utilisant le toluène comme solvant de l'acide undécylénique en présence d'un catalyseur, dans un dispositif utilisant successivement plusieurs colonnes de grande capacité (volume), dans lesquelles est introduit de l'HBr sous forme de gaz.

La réaction radicalaire d'hydrobromuration est opérée de préférence dans les solvants non polaires ou apolaires, c'est-à-dire de faible moment dipolaire (car les solvants polaires, à fort moment dipolaire, captent les radicaux libres) et avec des réactifs les plus purs possibles (car les impuretés détruisent les radicaux libres).

Pour pouvoir solubiliser l'HBr et les autres réactifs à la température de la réaction d'hydrobromuration, les procédés de l'art antérieur utilisent généralement des solvants, tels que benzène, toluène, méthylcyclohexane, trifluorotoluène (« BTF »), iso-octane, heptane. Les solvants aromatiques comme le benzène, toluène, xylène, ainsi que les solvants halogénés tels que le monochlorobenzène, sont généralement qualifiés de « bons solvants ». Par « bon solvant », on entend un solvant qui permet une miscibilité et solubilité des réactifs et des produits de réaction d'hydrobromuration à la température de la réaction d'hydrobromuration, et qui offre ainsi une meilleure réactivité au système. La miscibilité désigne usuellement la capacité de divers liquides à se mélanger. Si le mélange obtenu est homogène, les liquides sont qualifiés de miscibles au sens de l'invention. La solubilité d'un composé ionique ou moléculaire, appelé soluté, est la concentration maximale (en moles par litre) de ce composé que l'on peut dissoudre ou dissocier dans un solvant, à une température donnée.

En permettant la solubilité totale des réactifs et produits, un bon solvant évite également les bouchages dans l'installation. A l'inverse, les solvants tels que le cyclohexane, méthylcyclohexane, l'heptane sont généralement classés « mauvais solvants » dans ce type de procédé d'hydrobromuration.

A l'avenir, pour des raisons d'hygiène, sécurité et environnement, l'utilisation des solvants aromatiques et/ou halogénés devrait être évitée. D'autre part, les bons solvants susmentionnés ont comme autre inconvénient d'être généralement difficiles à prélever du système par évaporation.

La présente invention a donc pour but de fournir un procédé qui permet d'égaler, voire de dépasser, le rendement obtenue avec le(s) procédé(s) actuel(s), en utilisant tous types de solvants, y compris des « mauvais solvants » non aromatiques ni halogénés qui solubilisent difficilement les réactifs dans les procédés actuels.

Par ailleurs, les procédés d'hydrobromuration actuels avec colonne à garnissage imposent pour le recyclage de HBr, le recours à un système de type rebouilleur (stripping) qui entraine une forte dégradation de la teinte du produit bromé, c'est-à-dire conduit à un produit fortement coloré brun.

La présente invention a également pour but de trouver un procédé industriel continu permettant de maîtriser et d'optimiser les divers paramètres de la réaction d'hydrobromuration : mélange des réactifs, température de réaction, temps de séjour, quantité de solvant, débit des réactifs, recyclage des réactifs et/ou solvants, et permettant d'atteindre les spécifications imposées, notamment en taux de conversion et rendement, et d'améliorer la qualité du produit obtenu.

La Demanderesse a maintenant trouvé un tel procédé continu d'hydrobromuration caractérisé par un régime d'écoulement et un temps de séjour particuliers permettant de résoudre le problème précité.

La présente invention a donc pour objet, un procédé de synthèse d'un produit de formule R1-CHBr-CH2-R2, de préférence Br-(CH2)2-R2, par hydrobromuration en faisant réagir :

- un réactif de formule (I) R1-CH=CH-R2 dans laquelle R1, R2 identiques ou différents sont choisis parmi H ou un radical alkyle comportant de 1 à 30 atomes de carbone, de préférence de 1 à 20 atomes de carbone de préférence de 6 à 15 atomes de carbone, saturé ou insaturé, linéaire ou ramifié, fonctionnalisé ou non fonctionnalisé,

- avec de l'HBr en excès molaire par rapport au réactif de formule (I),
- en présence d'un initiateur radicalaire,
- et en présence optionnelle d'au moins un solvant,

ledit procédé étant caractérisé en ce qu'il comprend une étape **A** de mélange d'au moins le réactif I et de l'HBr, ces derniers arrivant chacun respectivement sous la forme d'un flux liquide dans un dispositif de mélange dA dans lequel règne un régime d'écoulement turbulent, de façon à atteindre à l'issue de cette étape A un taux de conversion compris dans la gamme de 10 à 100%, et en ce qu'il comprend en outre une étape B de poursuite spontanée de la réaction dans un dispositif dB dans lequel règne un régime d'écoulement transitoire ou laminaire, de façon à atteindre à l'issue de cette étape un taux de conversion supérieur à 90%.

Le réactif I (ou composé I) utilisé est sous forme liquide, soit fondu à une température supérieure à sa température de fusion, soit dissous dans un solvant. Le composé ou réactif I est de formule R1-CH=CH-R2, de préférence $CH_2$=CH-R2. On peut citer par exemple $CH_2$=CH-R'-COOH dans lequel R' est un radical alkyle, comportant de 1 à 29 atomes de carbone, de préférence de 1 à 19 atomes de carbone, de préférence de 6 à 14 atomes de carbone, saturé ou insaturé (alcène, alcyne), linéaire ou ramifié, fonctionnalisé (notamment par au moins une fonction acide, ester, nitrile, alcool et/ou aldéhyde) ou non fonctionnalisé. Ainsi, la présente invention vise en particulier la fabrication de produits $Br-(CH_2)_2$-R2, par exemple $Br-(CH_2)_2$-R'-COOH.

L'HBr utilisé dans le procédé de l'invention est sous forme liquide, notamment liquéfié sous sa pression de vapeur saturante ou en solution dans un solvant ou mélange de solvants tel que décrit plus bas. On peut notamment citer l'HBr commercialisé par L'AIR LIQUIDE ou par Air Products.

L'initiateur radicalaire est choisi parmi l'air, $O_2$, un peroxyde, un composé diazo, ou tout autre générateur de radicaux tel que des UV. Selon un mode de réalisation particulier du procédé de l'invention, l'initiateur radicalaire comprend de préférence de l'oxygène, préféré pour sa facilité de mise en oeuvre industrielle, sa stabilité, et son coût moindre par rapport aux autres types d'initiateurs. L'initiateur radicalaire peut être ajouté en amont du dispositif dA, par exemple ajouté au flux de HBr, ou bien directement dans le dispositif de mélange dA, ou encore en aval du dispositif dA, c'est-à-dire dans le flux comportant le mélange de HBr et de réactif I sortant de dA, ou encore à plusieurs endroits parmi ceux cités précédemment.

L'hydrobromuration selon le procédé de l'invention est opérée en milieu liquide homogène, ou bien en phase liquide-gaz, notamment dans le cas où l'initiateur radicalaire est injecté sous forme de gaz directement dans le dispositif dA, ou bien en aval de dA. De préférence, l'hydrobromuration est opérée en milieu liquide homogène.

Dans le cas où un solvant est utilisé, on utilise de préférence un bon solvant à la fois de l'HBr et du réactif 1 dissous. Le solvant utilisé peut être polaire ou apolaire, de préférence apolaire.

Lorsqu'on utilise un solvant dans le procédé de l'invention, celui-ci est choisi de préférence parmi : benzène, fluorobenzène, chlorobenzène, toluène, $\alpha,\alpha,\alpha$-trifluorotoluène, éthylbenzène, xylènes, cyclohexane, méthylcyclohexane, méthylcyclopentane, n-hexane, 2-méthylhexane, 3-méthylhexane, n-heptane, isooctane, tétrachloroéthylène, 1,1,1-trichloroéthane, dibromométhane, trichlorométhane, tétrachlorométhane, 1-bromopropane, carbonate de diméthyle, tétrahydrofurane (THF), 1,4-dioxane, 2-méthyltétrahydrofurane, tétrahydropyrane (THP), 1-propoxypropane, 1-ethoxybutane, 2-isopropoxypropane, acétonitrile, et leurs mélanges.

De préférence, le solvant comprend au moins un des solvants suivants : benzène, fluorobenzène, chlorobenzène, trifluorotoluène (« BTF »), éthylbenzène, toluène, xylène, cyclohexane, méthylcyclohexane, heptane, iso-octane, 1,1,1-trichloroéthane, dibromométhane, et leurs mélanges.

De préférence, le procédé de l'invention utilise au moins un solvant choisi parmi les solvants aliphatiques, tels que cyclohexane, méthylcyclohexane, heptane et leurs mélanges ; éventuellement en mélange avec au moins un autre solvant choisi parmi les solvants aromatiques tels que benzène, toluène, xylène, ou les solvants halogénés tels que monochlorobenzène, et leurs mélanges.

De manière avantageuse, les solvants éventuels arrivent dans le dispositif de mélange à une température comprise dans la gamme de -50°C à 20°C, de préférence de -40°C à 10°C, de préférence de -30°C à 0°C, de préférence de -30°C à -10°C. En effet, pour avoir HBr et le réactif I solubles dans le solvant, la température d'utilisation du solvant est de préférence comprise entre la + haute température de cristallisation des réactifs et la température limite de la réaction anti-markovnikov, c'est-à-dire dans la gamme de -50°C à 20°C. Le dispositif dA peut comprendre en outre un système de refroidissement pour compenser l'exothermie de la réaction d'hydrobromuration, et favoriser la réaction anti-Markovnikov.

Dans le dispositif de mélange dA règne un régime d'écoulement **turbulent.** On atteint à l'issue de cette étape A un taux de conversion compris dans la gamme de 10 à 100%. Avantageusement, le régime d'écoulement **turbulent** de l'étape **A** est caractérisé par un nombre de Reynolds > 1800, de préférence > 2000, de préférence **> 3000.** Le régime turbulent est réglé de manière connue par l'homme du métier, en adaptant la P entrante induite par le débit et la section des tuyaux (tube, dimensionnement) arrivant dans le dispositif de mélange dA ; et en forçant la diffusion des flux dans le dispositif, cette diffusion étant liée notamment au volume du dispositif de mélange dA.

Grâce au très bon mélange instantané initial permis par l'étape A, qui optimise la rencontre des réactifs, le procédé de l'invention est plus rapide que les procédés classiques utilisant un réacteur à colonne garnie.

Avantageusement, le temps de séjour de l'étape **A** est inférieur à 10 minutes, de préférence compris dans la gamme de 0,01 seconde à 10 minutes, de préférence compris dans la gamme de 0,01 seconde à 5 minutes, de préférence compris dans la gamme de 0,01 seconde à 1 minute, de préférence de 0,01 à 30 secondes, de préférence de 0,01 à 10 secondes, de préférence de 0,2 à 5 secondes, de préférence de 0,2 à 2,5 secondes, de préférence de 0,2 à 2 secondes, de préférence de 0,2 à 1 s.

Avantageusement, le temps de séjour de l'étape **A** est inférieur au temps de réaction.

Le fait d'initier la réaction très rapidement et de forcer les réactifs à se rencontrer au moyen du dispositif dA, permet leur mélange plus intime et plus rapide, et donc leur réaction, puis la réaction se propage spontanément. Le procédé de l'invention permet d'utiliser des « mauvais solvants » tels que définis plus haut, et tel que démontré dans les exemples ci-après.

Selon un mode de réalisation particulier, le taux de conversion à l'issue de l'étape A est compris dans la gamme de 10 à 99,99%, de préférence de 10 à 90%. Le procédé de l'invention comprend en outre une étape **B** de poursuite spontanée (et/ou de finition) de la réaction dans un dispositif **dB** dans lequel règne un régime d'écoulement **transitoire ou laminaire,** de façon à atteindre à l'issue de cette étape un taux de conversion supérieur à 90%, de préférence d'au moins 95%.

De préférence, le régime d'écoulement transitoire ou laminaire de l'étape **B** est caractérisé par un nombre de Reynolds **< 3000,** de préférence < 2000, de préférence < 1800, de préférence < 1300.

Avantageusement, les temps de séjour dans les dispositifs **dA** et **dB** sont tels que le taux de conversion à l'issue de l'étape **A** est compris dans la gamme de 10 à 90%, tandis que le taux de conversion à l'issue de l'étape **B** est compris dans la gamme de 90 à 100%.

Selon un mode de réalisation particulier du procédé de l'invention, le composé de formule (I), et le solvant éventuel arrivent dans le dispositif de mélange **dA** en un flux Fa, tandis que l'HBr, le solvant éventuel, et l'amorceur, arrive(nt) dans le dispositif de mélange **dA** en un deuxième flux Fb au cours de l'étape A, le rapport des flux Fa et Fb étant maintenu sensiblement constant, et le flux Fr résultant du mélange des flux Fa et Fb est dirigé depuis la sortie du dispositif de mélange **dA** dans le dispositif de finition **dB** de la réaction.

Selon un autre mode de réalisation particulier du procédé de l'invention, le composé de formule (I), l'amorceur et le solvant éventuel arrivent dans le dispositif de mélange **dA** en un flux Fa, tandis que l'HBr et le solvant éventuel, arrive(nt) dans le dispositif de mélange **dA** en un deuxième flux Fb au cours de l'étape A, le rapport des flux Fa et Fb étant maintenu sensiblement constant, et le flux Fr résultant du mélange des flux Fa et Fb est dirigé depuis la sortie du dispositif de mélange **dA** dans le dispositif de finition **dB** de la réaction.

Avantageusement, le dispositif **dA** comprend un dispositif de l'ordre du décimètre, du centimètre, du millimètre ou du micromètre, tel qu'un « micro-dispositif », notamment microréacteur ou micromélangeur.

Avantageusement le dispositif de mélange **dA** utilisé dans le procédé de la présente invention comprend au moins un réacteur et/ou au moins un mélangeur, ayant chacun :

- un volume interne total faible, généralement compris dans la gamme de 1 microlitre à 200 litres, de préférence de 1 microlitre à 100 litres, de préférence de 1 microlitre à 10 litres, de préférence de 1 microlitre à 1 litre, de préférence de 1 microlitre à 500 millilitres, de préférence de 0,1 ml à 200 ml, de préférence de 0,1 ml à 100 ml, et de façon plus préférée pour le réacteur de 10 à 60 ml ;
- une valeur du rapport entre sa surface et son volume internes supérieur à 50 $m^2m^{-3}$, de préférence supérieur à 500 $m^2m^{-3}$, de préférence supérieur à 1000 $m^2m^{-3}$ ;
- une dimension caractéristique, à savoir la distance la plus courte entre deux parois se faisant face, comprise dans la gamme de 1 $\mu$m à 1 m, de préférence 1 $\mu$m à 10 cm, de préférence de 1 $\mu$m à 1 cm, de préférence 0,5 mm à 5 mm et de façon plus préférée pour les microréacteurs de 1 à 3 mm.

Le dispositif dA peut notamment comprendre un ou plusieurs « microdispositif(s) », par exemple un ensemble de réacteur(s) et/ou mélangeur(s) microstructuré(s) monté(s) en série ou en parallèle.

Le dispositif de mélange **dA** a pour but premier de mettre en contact deux fluides, celui contenant le réactif I et celui contenant l'HBr, afin de les homogénéiser rapidement, de favoriser le transfert de matière et donc la réaction d'hydrobromuration. La géométrie et le type de mise en contact des fluides définissent la performance de l'appareil. Pour mélanger des fluides miscibles, la performance du dispositif de mélange **dA** est liée directement à la distance caractéristique entre les espèces, généralement déterminée par la géométrie du dispositif dA. Couplées avec les conditions opératoires du procédé selon l'invention, elles contrôlent les caractéristiques physiques du mélange. De vastes possibilités existent et dépendent des technologies de fabrication du dispositif retenues. À titre d'exemple, les modes de mise en contact de fluides les plus courants sont :

- contact de deux courants en T ou en Y. Si les dimensions du (micro)canal sont suffisamment petites, le processus de mélange par diffusion peut être rapide car la distance caractéristique du système est très faible. Cependant, pour mélanger rapidement dans des canaux plus grands, le débit traversant doit être suffisamment élevé afin d'induire des effets non-stationnaires dans l'écoulement ;
- multilamination par l'introduction de multiples courants de deux composants, souvent de manière interdigitale. Le temps de mélange est donc dépendant de la largeur de chaque filament de fluide, déterminée par la dimension des (micro)canaux. Dans le cas où la largeur de chaque canal est relativement importante (> 100 mm), le mélange par diffusion moléculaire étant peu efficace, le temps de mélange peut être diminué en couplant la multilamination avec le mécanisme de convergence hydrodynamique ;
- convergence hydrodynamique par la diminution de la distance diffusionnelle, normale à la direction de l'écoulement. En rétrécissant la largeur du (micro)canal de manière abrupte ou progressive, le mécanisme de convergence hydrodynamique permet de diminuer le temps de mélange en forçant les filaments de fluide par un orifice plus étroit, ce qui réduit la distance caractéristique pour la diffusion ;
- division et recombinaison des courants. Les fluides subissent des phases d'étirement, de découpage et de recombinaison, afin d'augmenter la surface de contact entre les lamelles de fluide et de diminuer leurs épaisseurs. Souvent, les écoulements générés avec ce mécanisme ont des caractéristiques turbulentes qui permettent un mélange rapide et efficace ;
- mécanismes de mélange chaotique lorsqu'une perturbation périodique est appliquée à l'écoulement.

On peut en outre citer à titre d'exemples de dispositifs utilisables dans le procédé de l'invention les mélangeurs à effet venturi.

L'écoulement turbulent favorisé dans le dispositif dA utilisé dans le procédé de l'invention est favorable au mélange puisqu'il permet de séparer à une vitesse exponentielle deux quantités infinitésimales de fluides initialement très proches et d'augmenter ainsi l'interface entre les espèces. Il peut être généré par une géométrie tri-dimensionnelle et périodique, ou lors d'une perturbation périodique dans le temps créée par une source externe (exemple l'injection périodique d'un courant normal à l'écoulement principal.

Des dispositifs utilisables pour le procédé de l'invention peuvent être les appareils commercialisés par IMM (par exemple Star Laminator, réacteurs à film tombant ou ruisselant), Ehrfeld BTS (par exemple de microjet mixer Impinging Jet, ou le réacteur MiProwa), LTF (Tangential flow), Mikroglas Chemtech, Corning (micro-réacteurs de verre), Zeton, Cytos, Velocys, les mélangeurs venturi, par exemple de type B3 ; pour des applications de mélange de fluides miscibles. Les verres utilisables pour fabriquer ces microréacteurs sont tous les types de verre tels que les verres borosilicates (Pyrex®, par exemple), les verres sodocalciques, les verres au plomb, les verres de silice ou les vitrocéramiques. On peut bien entendu choisir pour le dispositif tout autre matériau que le verre, dès l'instant qu'il est compatible avec le milieu réactionnel.

Avantageusement, l'étape A de mélange des réactifs est conduite dans des conditions de débits et de pression, tels qu'au sein du dispositif de mélange **dA** les fluides réactifs circulant présentent de faibles temps caractéristiques, à savoir un temps de séjour inférieur à 10 minutes, de préférence compris dans la gamme de 0,01 seconde à 5 minutes, de préférence compris dans la gamme de 0,01 seconde à 1 minute, de préférence de 0,01 à 30 secondes, de préférence de 0,01 à 10 secondes, de préférence de 0,2 à 5 secondes, de préférence de 0,2 à 2,5 secondes, de préférence de 0,2 à 2 secondes, de préférence de 0,2 à 1 s.

De préférence, lors de l'étape **A,** la température d'entrée du composé I dans le dispositif de mélange **dA** est comprise dans la gamme de 0 à 100°C, de préférence de 0 à 80°C, étant entendu que si le composé I entre seul (sans solvant), il est à une température supérieure à la température de fusion Tf du composé I, de préférence supérieure à Tf + 30°C, de préférence supérieure à Tf + 50°C, tandis que s'il entre dilué dans un solvant, ledit mélange de composé I et de solvant entre dans le dispositif dA à une température comprise dans la gamme de 0 à 50°C.

Avantageusement, lors de l'étape **A,** la température d'entrée de HBr, lorsqu'il entre dilué dans un solvant, est comprise dans la gamme de -50°C à 50°C, de préférence de -50°C à 0°C.

De préférence, le réactif HBr est en excès d'au moins 0,1% molaire, de préférence de 0,1 à 100% molaire, de préférence de 0,1 à 99,9% molaire, de préférence en excès compris dans la gamme de 1 à 50% molaire par rapport au réactif de formule (I).

Avantageusement, le ratio en poids HBr/solvant total éventuel est compris dans la gamme de 1 à 30%, de préférence de 2 à 25%, de préférence de 2 à 20%, de préférence de 4 à 15%.

Avantageusement, le ratio en poids réactif I/solvant total, lorsqu'un solvant est utilisé, est compris dans la gamme de 1 à 80%, de préférence de 1 à 70%, de préférence de 1 à 60%, de préférence de 1 à 50%, de préférence de 1 à 40%, de préférence de 2 à 25%, de préférence de 4 à 20%. Un avantage important du procédé selon l'invention est qu'il permet d'utiliser moins de solvant que les procédés de l'art antérieur.

Le ratio amorceur radicalaire / HBr est de préférence compris dans la gamme de 0,01 à 80%, de préférence de 0,05 à 70%, de préférence de 0,1 à 60%, voire de 0,1 à 50%.

De préférence, l'hydrobromuration est conduite à une température comprise dans la gamme de -50 à 50°C, de préférence de -20 à 20°C. Avantageusement, la réaction d'hydrobromuration est opérée au moins partiellement (c'est-à dire sur au moins une partie de la réaction) en mode adiabatique. Le procédé de l'invention permet ainsi une importante économie d'énergie.

De préférence, à l'issue de l'étape **A,** la température de sortie du produit du dispositif dA est supérieure à la température de cristallisation du produit, de préférence comprise dans la gamme de -50°C à 50°C, de préférence de -40°C à 40°C, de préférence de -30°C à 30°C, de préférence de -30°C à 20°C.

De préférence, la température de sortie du produit du dispositif dA ne doit pas dépasser 50°C pour favoriser une réaction Karash (c'est-à-dire limiter ou empêcher la réaction Markovnikov), et obtenir ainsi par exemple l'acide 11-Bromoundécanoïque au lieu du 10-Bromo. Mais à l'inverse, il est préférable que la température de sortie du produit du dispositif dA ne soit pas inférieure à -50°C car on risque de cristalliser ou figer et de bloquer le flux sortant.

Avantageusement, le procédé de l'invention n'utilise pas de catalyseur d'hyrobromuration. Bien entendu, il est tout de même possible d'utiliser un catalyseur, choisi parmi les catalyseurs connus pour ce type de réaction.

Au terme de l'étape de mélange **A** et/ou au terme de l'étape de finition **B,** on recueille un mélange de produit, de solvant éventuel et d'HBr résiduel, ledit procédé pouvant comprendre en outre une étape **C** de séparation et de récupération du produit, et le recyclage éventuel de l'HBr et/ou du solvant vers le dispositif de mélange **dA.**

La présente invention a également pour objet la fabrication de l'acide 11-bromoundécanoïque à partir de l'acide undécylènique, au moyen du procédé tel que défini ci-dessus. Avantageusement, le procédé de l'invention comprend en outre une étape d'ammonolyse (ou amination) pour former un composé de formule $NH_2\text{-}(CH_2)_n\text{-}R_2$, tel que l'acide 11-aminoundécanoïque. Avantageusement, le procédé de l'invention comprend en outre une étape de synthèse de polyamide par polymérisation du composé de formule $NH_2\text{-}(CH_2)_n\text{-}R_2$.

Exemples

Le procédé de l'invention est illustré par les exemples ci-après.

Exemples des essais 1 à 4 (selon l'invention)

Dans les exemples suivants, on teste différents solvants indiqués dans le tableau 1. Dans ces exemples, le mode opératoire est le même que celui décrit pour l'Essai 4 du tableau 1, utilisant un micro-mélangeur.

Description du micro-mélangeur : deux flux sont introduits suivant quatre jets latéraux Ja (2 jets opposés en face à face) et Jb (2 jets opposés en face à face), Ja étant la solution de C11 (éventuellement fondu) et Jb le solvant contenant l'HBr. Les jets sont inclinés chacun d'un angle de 90° par rapport à la verticale formée par ces quatre jets. La chambre de mélange a un volume de 0.32 ml.

Essai 4 (selon l'invention) : On prépare une solution avec 5,03 g d'acide bromhydrique gazeux dans 38,5 g de méthylcyclohexane, en envoyant un courant d'HBr dans le solvant refroidi à -20°C. En parallèle, on prépare une solution avec 352 g d'acide undécylénique dans 462 g de méthylcyclohexane dans laquelle on fait traverser un courant d'oxygène. Cette dernière solution est maintenue à 20°C. Les deux solutions sont ensuite envoyées (Débit HBr/ MeCHx = 50 ml/min, Débit C11/MeCHx = 25 ml/min) dans un micro-mélangeur qui permet un mélange intime des deux flux de liquide. La solution sortante de ce procédé est composée d'acide 11-bromoundécanoïque, de méthylcyclohexane et d'HBr résiduel. Cette solution est plongée dans un réceptacle contenant de l'eau afin d'éliminer l'HBr résiduel. La phase organique est lavée trois fois avec 500 mL d'eau puis évaporée sous pression réduite de manière à éliminer le méthylcyclohexane.

Les résultats obtenus sont :

Conversion = 99,9%
Rendement = 95,0%

Expression des résultats :

Le rendement (ou pourcentage de produit P, ici %Br11) est déterminé par la formule suivante :

$$\% \, P = \frac{\text{Surface du produit P}}{\text{Surface totale}} \times 100$$

Surface = aire ou intensité du pic du chromatogramme mesuré par chromatographie gazeuse (GC)

Dans le cas de la réaction d'hydrobromuration de l'acide undécylénique, on déterminera particulièrement :

- le pourcentage d'acide undécylénique (%C11) résiduel
- le pourcentage d'acide bromo-10 undécanoïque (%Br10)
- le pourcentage d'acide bromo-9 undécanoïque (%Br9)
- le pourcentage d'acide hydroxy-11 undécanoïque (%C11-OH)
- le pourcentage d'acide bromo -11 undécanoïque (%Br11)

La Surface totale dans la formule ci-dessus représente la somme des pics : C11 résiduel, C11-OH, Br9, Br10, Br11 du mélange réactionnel final.

Le taux de conversion C de l'acide undécylénique est déterminé par la formule suivante : C = 100 - % C11 de départ.

Comparatifs des essais 1 à 4 (utilisant une colonne)

Dans les comparatifs, on teste différents solvants indiqués dans le tableau 1, mais en utilisant le procédé et le dispositif de l'art antérieur (colonne), selon les conditions opératoires suivantes décrites dans le cas de l'essai 2 (comparatif) : Essai 2 (comparatif) :

- le solvant est un mélange cyclohexane/benzène 70/30 v/v,
- 15% massique d'acide undécylénique (« C11 ») dissout dans les solvants,
- colonne régulée à 0°C en partie haute et 10°C partie basse,
- température d'entrée C11/solvant = -12,5°C,
- Débit C11/solvant = 80 g/min
- Débit HBr = 6,32 g/min, soit un excès molaire de 20% par rapport au C11
- Débit air = 0,196 l/min
- Débit H2 = 0,184 l/min
  Rendement = 95,0%

Le tableau 1 suivant compare les essais 1 à 4 (différents solvants) réalisés avec colonne (comparatifs) ou avec micro-mélangeur (selon l'invention) :

*Tableau 1*

| | Solvant | | | | Rendement en Bromo 11 | |
| | "Bon solvant" | | "Mauvais solvant" | | | |
| Essais | Benzène | MCB (monochlorobenzène) | Cyclohexane | méthylcyclohexane | Comparatifs (Colonne) | Invention (Micro-mélangeur) |
|---|---|---|---|---|---|---|
| 1 | | 100% | | | 95,7% | 95,9% |
| 2 | 30% | | 70% | | 95,0% | 95,3% |
| 3 | | | 80% | 20% | 94,0% | 95,0% |
| 6 | | | | 100% | 93,6% | 95,0% |

Comme le montrent les essais du Tableau 1, lorsqu'on remplace le benzène par un « mauvais solvant » dans les procédés existants, on perd 1% de rendement en 11-Bromo (95% avec du benzène, 94% en colonne avec le meilleur des mauvais solvants). De manière surprenante, dans le procédé de l'invention, le même mauvais solvant permet au contraire un niveau de rendement rattrapé (95%) voire meilleur en 11-Bromo (pour une même réaction d'hydrobromuration). De façon inattendue, le meilleur gap (augmentation) de rendement est observé dans le cas des "mauvais solvants" avec le procédé de l'invention.

Exemple 7

Dans ces essais le micro mélangeur a été remplacé par un mélangeur de type Venturi.
Description du mélangeur venturi : un venturi en verre de 11 cm de longueur est alimenté par deux tuyaux de diamètre intérieur 4 mm et de diamètre extérieur 6 mm.
Un mélange cyclohexane/méthylcyclohexane (« CHx/MeCHx ») 80/20 en volume est envoyé dans une colonne d'absorption via une pompe péristaltique à un débit de 150 ml/min. HBr gazeux est envoyé dans le milieu de la colonne d'absorption à un débit de 9.7 g/min soit un excès molaire de 11% par rapport au C11. L'acide C11 pur est envoyé à 50°C dans le dispositif de mélange (mélangeur venturi) à un débit de 19.9 g/min représentant une concentration massique de 14.5% par rapport à la totalité du solvant. La solution organique saturée en HBr est envoyée dans le dispositif de mélange via une pompe à engrenages à un débit de 150 ml/min. L'oxygène est injecté dans le circuit juste après la pompe à engrenage avec un système d'injection : tuyau capillaire plongeant dans le liquide à un débit de 0.04 nL/min. Le dispositif de mélange est suivi d'un tuyau en PFA (Perfluoroalkoxy) d'un diamètre intérieur de 4 mm et d'une longueur de 4 m.

| Débit solvant ml/min | Débit Oxyène nl/min | Débit C11 pur g/min | Débit HBr g/min | T°C Pied de colonne | T°C Pied de colonne | Conversion % | Bromo 11 % |
|---|---|---|---|---|---|---|---|
| 150 | 0.040 | 19.9 | 9.7 | -20 | -17 | 99.94 | 95.00 |

Le taux de conversion et le rendement en bromo 11, obtenus avec le mélangeur Venturi sont identiques à ceux obtenus avec le micro mélangeur.

Exemple 8

Dans cet essai le micro mélangeur a été remplacé par un simple T en verre, « mélangeur en té ».
Description du mélangeur en té utilisé: Le té en verre de 5 cm de longueur est alimenté par deux tuyaux de diamètre intérieur 4 mm et de diamètre extérieur 6 mm. Ces tuyaux forment entre eux un angle de 90°.
Le mélange CHx/MeCHx 80/20 volume est envoyé dans une colonne d'absorption via une pompe péristaltique à un débit de 150 ml/min. HBr gazeux est envoyé dans le milieu de la colonne d'absorption à un débit de 9.7 g/min soit un excès molaire de 11% par rapport au C11. L'acide C11 pur est envoyé à 50°C dans le mélangeur T à un débit de 19.9 g/min représentant une concentration massique de 14.5% par rapport à la totalité du solvant. La solution organique saturée en HBr est envoyée dans le dispositif de mélange T via une pompe à engrenages à un débit de 150 ml/min. L'oxygène est injecté dans le circuit juste après la pompe à engrenage avec le système d'injection : tuyau capillaire plongeant dans le liquide à un débit de 0.04 nL/min. Le dispositif de mélange est suivi d'un tuyau en PFA d'un diamètre intérieur de 4 mm et d'une longueur de 4 m.

| Débit solvant ml/min | Débit Oxyène nl/min | Débit C11 pur g/min | Débit HBr g/min | T°C Pied de colonne | T°C Entrée mélangeur | T°C Sortie serpentin | Conversion % | Bromo 11 % |
|---|---|---|---|---|---|---|---|---|
| 150 | 0.040 | 19.9 | 9.7 | -20 | -17 | 26 - 27°C | 99.95 | 94.80 |

Le taux de conversion et le rendement en bromo 11, obtenus avec ce T sont identiques à ceux obtenus avec le micro mélangeur.

**Revendications**

1. Procédé de synthèse d'un produit de formule R1-CHBr-CH$_2$-R2, de préférence Br-(CH$_2$)$_2$-R2, par hydrobromuration en faisant réagir :

   - un réactif de formule (I) R1-CH=CH-R2 dans laquelle R1, R2 identiques ou différents sont choisis parmi H ou un radical alkyle comportant de 1 à 30 atomes de carbone, saturé ou insaturé, linéaire ou ramifié, fonctionnalisé ou non fonctionnalisé,
   - avec de l'HBR en excès molaire par rapport au réactif de formule (I),
   - en présence d'un initiateur radicalaire,
   - et en présence optionnelle d'au moins un solvant,

   ledit procédé étant **caractérisé en ce qu'**il comprend une étape **A** de mélange d'au moins le réactif I et de l'HBR, ces derniers arrivant chacun sous la forme d'un flux liquide dans un dispositif de mélange **dA** dans lequel règne un régime d'écoulement **turbulent,** de façon à atteindre à l'issue de cette étape A un taux de conversion compris dans la gamme de 10 à 100%, et **en ce qu'**il comprend en outre une étape **B** de poursuite spontanée de la réaction dans un dispositif **dB** dans lequel règne un régime d'écoulement **transitoire ou laminaire,** de façon à atteindre à l'issue de cette étape un taux de conversion supérieur à 90%.

2. Procédé selon l'une quelconque des revendications précédentes, **caractérisé en ce que** les temps de séjour dans les dispositifs **dA** et **dB** sont tels que le taux de conversion à l'issue de l'étape **A** est compris dans la gamme de 10 à 90%, tandis que le taux de conversion à l'issue de l'étape **B** est compris dans la gamme de 90 à 100%.

3. Procédé selon l'une quelconque des revendications 1 ou 2, dans lequel le régime d'écoulement **turbulent** de l'étape **A** est **caractérisé par** un nombre de Reynolds > 1800.

4. Procédé selon l'une quelconque des revendications précédentes, dans lequel le temps de séjour de l'étape **A** est inférieur à 10 minutes.

5. Procédé selon l'une quelconque des revendications précédentes, dans lequel le temps de séjour de l'étape **A** est inférieur au temps de réaction.

6. Procédé selon l'une quelconque des revendications précédentes, dans lequel le régime d'écoulement transitoire ou laminaire de l'étape **B** est **caractérisé par** un nombre de Reynolds **< 3000.**

7. Procédé selon l'une quelconque des revendications précédentes, **caractérisé en ce que** l'initiateur radicalaire est choisi parmi l'air, O$_2$, peroxyde, composé diazo, ou tout autre générateur de radicaux tel que des UV.

8. Procédé selon l'une quelconque des revendications précédentes, **caractérisé en ce que** le réactif (I) utilisé est sous forme liquide, soit fondu à une température supérieure à sa température de fusion, soit dissous dans un solvant.

9. Procédé selon l'une quelconque des revendications précédentes, **caractérisé en ce que** l'hydrobromuration est opérée en milieu liquide homogène.

10. Procédé selon l'une quelconque des revendications précédentes, **caractérisé en ce que** l'HBr utilisé est sous forme liquide, liquéfié sous sa pression de vapeur saturante ou en solution dans un solvant.

11. Procédé selon l'une quelconque des revendications précédentes, **caractérisé en ce que** le solvant comprend au moins un des solvants suivants : benzène, fluorobenzène, chlorobenzène, trifluorotoluène (« BTF »), éthylbenzène, toluène, xylène, cyclohexane, méthylcyclohexane, heptane, iso-octane, 1,1,1-trichloroéthane, dibromométhane, et leurs mélanges.

12. Procédé selon l'une quelconque des revendications précédentes, **caractérisé en ce qu'**il utilise au moins un solvant choisi parmi les solvants aliphatiques, tels que cyclohexane, méthylcyclohexane, heptane et leurs mélanges ; éventuellement en mélange avec au moins un autre solvant choisi parmi les solvants aromatiques, tels que benzène, toluène, xylène, ou les solvants halogénés tels que monochlorobenzène, et leurs mélanges.

13. Procédé selon l'une quelconque des revendications précédentes, **caractérisé en ce que** les solvants éventuels arrivent dans le dispositif de mélange à une température comprise dans la gamme de -50°C à 20°C.

14. Procédé selon l'une quelconque des revendications précédentes, dans lequel le composé de formule (I), et le solvant éventuel arrivent dans le dispositif de mélange **dA** en un flux Fa, tandis que l'HBr, le solvant éventuel et l'amorceur arrivent dans le dispositif de mélange **dA** en un deuxième flux Fb au cours de l'étape A, le rapport des flux Fa et Fb étant maintenu sensiblement constant, et le flux Fr résultant du mélange des flux Fa et Fb est dirigé depuis la sortie du dispositif de mélange **dA** dans le dispositif de finition **dB** de la réaction.

15. Procédé selon l'une quelconque des revendications précédentes, dans lequel le dispositif de **dA** comprend au moins un réacteur et/ou au moins un mélangeur, ayant chacun :

    - un volume interne total faible, généralement compris dans la gamme de 1 microlitre à 200 litres,
    - une valeur du rapport entre sa surface et son volume internes supérieur à 50 m$^2$m$^{-3}$,
    - une dimension caractéristique, à savoir la distance la plus courte entre deux parois se faisant face, comprise dans la gamme de 1 $\mu$m à 1 m.

16. Procédé selon l'une quelconque des revendications précédentes, **caractérisé en ce que** l'étape de mélange des réactifs est conduite dans des conditions de débits et de pression telles qu'au sein du dispositif de mélange dA les fluides réactifs circulant présentent de faibles temps caractéristiques, à savoir un temps de séjour inférieur à 10 minutes, de préférence compris dans la gamme de 0,01 seconde à 5 minutes.

17. Procédé selon l'une quelconque des revendications précédentes, **caractérisé en ce que** lors de l'étape **A,** la température d'entrée du composé I dans le dispositif de mélange **dA** est comprise dans la gamme de 0 à 100°C, étant entendu que si le composé I entre seul, il est à une température supérieure à la température de fusion Tf du composé I, tandis que s'il entre dilué dans un solvant, ledit mélange de composé I et de solvant entre dans dA à une température comprise dans la gamme de 0 à 50°C.

18. Procédé selon l'une quelconque des revendications précédentes, **caractérisé en ce que** lors de l'étape **A,** la température d'entrée de HBr lorsqu'il entre dilué dans un solvant, est comprise dans la gamme de -50°C à 50°C.

19. Procédé selon l'une quelconque des revendications précédentes, **caractérisé en ce que** le réactif HBr est en excès d'au moins 0,1% molaire, par rapport au réactif de formule (I).

20. Procédé selon l'une quelconque des revendications précédentes, **caractérisé en ce que** le ratio en poids HBr/solvant total éventuel est compris dans la gamme de 1 à 30%.

21. Procédé selon l'une quelconque des revendications précédentes, **caractérisé en ce que** le ratio en poids réactif I/solvant total éventuel est compris dans la gamme de 1 à 80%.

22. Procédé selon l'une quelconque des revendications précédentes, dans lequel le ratio amorceur radicalaire / HBr est compris dans la gamme de 0,01 à 80%.

23. Procédé selon l'une quelconque des revendications précédentes **caractérisé en ce que** l'hydrobromuration est conduite à une température comprise dans la gamme de -50 à 50°C.

24. Procédé selon l'une quelconque des revendications précédentes, dans lequel la réaction d'hydrobromuration est opérée au moins partiellement en mode adiabatique.

25. Procédé selon l'une quelconque des revendications précédentes, **caractérisé en ce qu'**à l'issue de l'étape **A**, la température de sortie du produit du dispositif dA est supérieure à la température de cristallisation du produit, de préférence comprise dans la gamme de -50°C à 50°C.

26. Procédé selon l'une quelconque des revendications précédentes dans lequel, au terme de l'étape de mélange **A** et/ou au terme de l'étape de poursuite spontanée **B,** on recueille un mélange de produit, de solvant éventuel et d'HBr résiduel, ledit procédé comprenant en outre une étape C de séparation et de récupération du produit, et le recyclage éventuel de l'HBr et/ou du solvant vers le dispositif de mélange A.

27. Procédé selon l'une quelconque des revendications précédentes, comprenant la fabrication de l'acide 11-bromoundécanoïque à partir de l'acide undécylènique.

28. Procédé selon l'une quelconque des revendications précédentes comprenant en outre une étape d'ammonolyse pour former un composé de formule $NH_2-(CH_2)_n-R$, tel que l'acide 11-aminoundécanoïque.

29. Procédé selon la revendication précédente comprenant en outre une étape de synthèse de polyamide par polymérisation du composé de formule $NH_2-(CH_2)_n-R$.

## Patentansprüche

1. Verfahren zur Synthese eines Produkts der Formel $R1-CHBr-CH_2-R2$, vorzugsweise $Br-(CH_2)_2-R2$, durch Hydrobromierung durch Umsetzung von

   - einem Reagens der Formel (I) $R1-CH=CH-R2$, in der R1 und R2 gleich oder verschieden sind und aus H oder einem funktionalisierten oder nicht funktionalisierten, linearen oder verzweigten, gesättigten oder ungesättigten Alkylrest ausgewählt sind,
   - mit HBr in molare im Überschuss, bezogen auf das Reagens der Formel (I),
   - in Gegenwart eines Radikalinitiators,
   - und gegebenenfalls in Gegenwart mindestens eines Lösungsmittels,

   wobei das Verfahren **dadurch gekennzeichnet ist, dass** es einen Schritt **A** des Mischens von mindestens dem Reagens I und dem HBr umfasst, wobei Letztere jeweils in Form eines flüssigen Stroms in einer Mischvorrichtung **dA** ankommen, in der ein **turbulentes** Strömungsregime herrscht, so dass am Ende dieses Schritts A ein Umsatzgrad im Bereich von 10 bis 100 % erreicht wird, und dass es außerdem einen Schritt **B** der spontanen Fortsetzung der Reaktion in einer Vorrichtung **dB,** in der ein **transitorisches** oder **laminares** Strömungsregime herrscht, umfasst, so dass am Ende dieses Schritts ein Umsatzgrad von mehr als 90 % erreicht wird.

2. Verfahren nach einem der vorhergehenden Ansprüche, **dadurch gekennzeichnet, dass** die Verweilzeiten in den Vorrichtungen **dA** und **dB** derart beschaffen sind, dass der Umsatzgrad am Ende von Schritt **A** im Bereich von 10 bis 90 % liegt, während der Umsatzgrad am Ende von Schritt **B** im Bereich von 90 bis 100 % liegt.

3. Verfahren nach einem der Ansprüche 1 oder 2, wobei das **turbulente** Strömungsregime von Schritt **A** durch eine Reynolds-Zahl > 1800 gekennzeichnet ist.

4. Verfahren nach einem der vorhergehenden Ansprüche, wobei die Verweilzeit von Schritt **A** kleiner als 10 Minuten ist.

5. Verfahren nach einem der vorhergehenden Ansprüche, wobei die Verweilzeit von Schritt **A** kleiner als die Reaktionszeit ist.

6. Verfahren nach einem der vorhergehenden Ansprüche, wobei das transitorische oder laminare Strömungsregime von Schritt **B** durch eine Reynolds-Zahl **< 3000** gekennzeichnet ist.

7. Verfahren nach einem der vorhergehenden Ansprüche, **dadurch gekennzeichnet, dass** der Radikalinitiator aus Luft, $O_2$, Peroxid, einer Diazoverbindung oder einem anderen Radikalbildner wie UV-Strahlung ausgewählt wird.

8. Verfahren nach einem der vorhergehenden Ansprüche, **dadurch gekennzeichnet, dass** das verwendete Reagens (I) in flüssiger Form vorliegt, entweder schmelzflüssig bei einer Temperatur oberhalb seines Schmelzpunkts oder in einem Lösungsmittel gelöst.

9. Verfahren nach einem der vorhergehenden Ansprüche, **dadurch gekennzeichnet, dass** die Hydrobromierung in einem homogenen flüssigen Medium durchgeführt wird.

10. Verfahren nach einem der vorhergehenden Ansprüche, **dadurch gekennzeichnet, dass** das verwendete HBr in flüssiger Form vorliegt, unter seinem Sättigungsdampfdruck verflüssigt oder in Lösung in einem Lösungsmittel.

11. Verfahren nach einem der vorhergehenden Ansprüche, **dadurch gekennzeichnet, dass** das Lösungsmittel mindestens eines der folgenden Lösungsmittel umfasst: Benzol, Fluorbenzol, Chlorbenzol, Trifluortoluol ("BTF"), Ethylbenzol, Toluol, Xylol, Cyclohexan, Methylcyclohexan, Heptan, Isooctan, 1,1,1-Trichlorethan, Dibrommethan und Mischungen davon.

12. Verfahren nach einem der vorhergehenden Ansprüche, **dadurch gekennzeichnet, dass** mindestens ein Lösungsmittel verwendet wird, das aus aliphatischen Lösungsmitteln wie Cyclohexan, Methylcyclohexan, Heptan und Mischungen davon ausgewählt wird; gegebenenfalls als Mischung mit mindestens einem anderen Lösungsmittel, das aus aromatischen Lösungsmitteln wie Benzol, Toluol oder Xylol oder halogenierten Lösungsmitteln wie Monochlorbenzol und Mischungen davon ausgewählt wird.

13. Verfahren nach einem der vorhergehenden Ansprüche, **dadurch gekennzeichnet, dass** die fakultativen Lösungsmittel bei einer Temperatur im Bereich von -50 °C bis 20 °C in der Mischvorrichtung ankommen.

14. Verfahren nach einem der vorhergehenden Ansprüche, wobei im Lauf von Schritt A die Verbindung der Formel (I) und das fakultative Lösungsmittel in einem Strom Fa in der Mischvorrichtung **dA** ankommen, während das HBr, das fakultative Lösungsmittel und der Initiator in einem zweiten Strom Fb in der Mischvorrichtung **dA** ankommen, wobei das Verhältnis der Ströme Fa und Fb weitgehend konstant gehalten wird, und der sich aus dem Mischen der Ströme Fa und Fb ergebende Strom Fr vom Ausgang der Mischvorrichtung **dA** in die Reaktionsfertigstellungsvorrichtung **dB** geleitet wird.

15. Verfahren nach einem der vorhergehenden Ansprüche, wobei die Vorrichtung **dA** mindestens einen Reaktor und/oder mindestens einen Mischer umfasst, jeweils mit:

- einem kleinen Gesamtinnenvolumen, im Allgemeinen im Bereich von 1 Mikroliter bis 200 Liter,
- einen Wert des Verhältnisses zwischen seiner Oberfläche und seinem Innenvolumen von mehr als 50 $m^2m^{-3}$,
- einer charakteristischen Abmessung, nämlich dem kürzesten Abstand zwischen zwei einander gegenüberliegenden Wänden im Bereich von 1 $\mu$m bis 1 m.

16. Verfahren nach einem der vorhergehenden Ansprüche, **dadurch gekennzeichnet, dass** der Schritt des Mischens der Reagentien unter solchen Strömungsraten- und Druckbedingungen durchgeführt wird, dass in der Mischvorrichtung **dA** die strömenden reaktiven Fluide kurze charakteristische Zeiten, nämlich eine Verweilzeit von weniger als 10 Minuten, vorzugsweise im Bereich von 0,01 Sekunden bis 5 Minuten, aufweisen.

17. Verfahren nach einem der vorhergehenden Ansprüche, **dadurch gekennzeichnet, dass** in Schritt **A** die Temperatur, bei der die Verbindung I in die Mischvorrichtung **dA** eintritt, im Bereich von 0 bis 100 °C liegt, mit der Maßgabe, dass die Verbindung I dann, wenn sie alleine eintritt, bei einer Temperatur oberhalb der Schmelztemperatur Tf der Verbindung I vorliegt, wohingegen dann, wenn sie in einem Lösungsmittel verdünnt eintritt, die Mischung von Verbindung I und Lösungsmittel bei einer Temperatur im Bereich von 0 bis 50 °C in dA eintritt.

18. Verfahren nach einem der vorhergehenden Ansprüche, **dadurch gekennzeichnet, dass** in Schritt **A** die Eintrittstemperatur von HBr dann, wenn es in einem Lösungsmittel verdünnt eintritt, im Bereich von -50 °C bis 50 °C liegt.

19. Verfahren nach einem der vorhergehenden Ansprüche, **dadurch gekennzeichnet, dass** das HBr-Reagens in einem Überschuss von mindestens 0,1 Mol-%, bezogen auf das Reagens der Formel (I), vorliegt.

20. Verfahren nach einem der vorhergehenden Ansprüche, **dadurch gekennzeichnet, dass** das Gewichtsverhältnis von HBr zu fakultativem gesamtem Lösungsmittel im Bereich von 1 bis 30 % liegt.

21. Verfahren nach einem der vorhergehenden Ansprüche, **dadurch gekennzeichnet, dass** das Gewichtsverhältnis von Reagens I zu fakultativem gesamtem Lösungsmittel im Bereich von 1 bis 80 % liegt.

22. Verfahren nach einem der vorhergehenden Ansprüche, wobei das Verhältnis von Radikalinitiator zu HBr im Bereich von 0,01 bis 80 % liegt.

23. Verfahren nach einem der vorhergehenden Ansprüche, **dadurch gekennzeichnet, dass** die Hydrobromierung bei einer Temperatur im Bereich von -50 bis 50 °C durchgeführt wird.

24. Verfahren nach einem der vorhergehenden Ansprüche, wobei die Hydrobromierungsreaktion zumindest teilweise in adiabatischer Fahrweise durchgeführt wird.

25. Verfahren nach einem der vorhergehenden Ansprüche, **dadurch gekennzeichnet, dass** am Ende von Schritt **A** die Temperatur, bei der das Produkt aus der Vorrichtung dA austritt, höher ist als die Kristallisationstemperatur des Produkts und vorzugsweise im Bereich von -50 °C bis 50 °C liegt.

26. Verfahren nach einem der vorhergehenden Ansprüche, bei dem man am Ende des Mischschritts **A** und/oder am Ende des Schritts der spontanen Fortsetzung **B** ein Gemisch von Produkt, fakultativem Lösungsmittel und restlichem HBr sammelt, wobei das Verfahren außerdem einen Schritt **C** der Abtrennung und Gewinnung des Produkts und die fakultative Rezyklierung des HBr und/oder des Lösungsmittels zur Mischvorrichtung A umfasst.

27. Verfahren nach einem der vorhergehenden Ansprüche, umfassend die Herstellung von 11-Bromundecansäure aus Undecylensäure.

28. Verfahren nach einem der vorhergehenden Ansprüche, außerdem umfassend einen Schritt der Ammonolyse zur Bildung einer Verbindung der Formel $NH_2$-$(CH_2)_n$-R, wie 11-Aminoundecansäure.

29. Verfahren nach dem vorhergehenden Anspruch, außerdem umfassend einen Schritt der Synthese von Polyamid durch Polymerisation der Verbindung der Formel $NH_2$-$(CH_2)_n$-R.


**Claims**

1. Process for synthesizing a product of formula R1-CHBr-CH$_2$-R2, preferably Br-$(CH_2)_2$-R2 by hydrobromination by reacting:

   - a reagent of formula (I) R1-CH=CH-R2 in which R1 and R2, which may be identical or different, are chosen from H or an alkyl radical comprising from 1 to 30 carbon atoms, which is saturated or unsaturated, linear or branched, and functionalized or non-functionalized,
   - with a molar excess of HBr relative to the reagent of formula (I),
   - in the presence of a radical initiator,
   - and optionally in the presence of at least one solvent,

   said process being **characterized in that** it comprises a step **A** of mixing at least the reagent I and the HBr, each of the latter arriving respectively in the form of a liquid stream in a mixing device **dA,** wherein a **turbulent** flow system prevails, so as to reach, at the end of this step A, a degree of conversion included in the range of from 10% to 100%, and **in that** it also comprises a step **B** of spontaneous continuation of the reaction in a device **dB** in which a **transient** or **laminar** flow system prevails, so as to reach, at the end of this step, a degree of conversion of greater than 90%.

2. Process according to any one of the preceding claims, **characterized in that** the residence times in the devices **dA** and **dB** are such that the degree of conversion at the end of step **A** is included in the range of from 10% to 90%, while the degree of conversion at the end of step **B** is included in the range of from 90% to 100%.

3. Process according to either one of Claims 1 and 2, wherein the **turbulent** flow system of step **A** is **characterized by** a Reynolds number > 1800.

4. Process according to any one of the preceding claims, wherein the residence time of step **A** is less than 10 minutes.

5. Process according to any one of the preceding claims, wherein the residence time of step **A** is less than the reaction time.

6. Process according to any one of the preceding claims, wherein the transient or laminar flow system of step **B** is **characterized by** a Reynolds number **< 3000.**

7. Process according to any one of the preceding claims, **characterized in that** the radical initiator is chosen from air, O$_2$, peroxide, a diazo compound, or any other radical generator such as UV radiation.

8. Process according to any one of the preceding claims, **characterized in that** the reagent (I) used is in liquid form, either melted at a temperature above its melting point, or dissolved in a solvent.

9. Process according to any one of the preceding claims, **characterized in that** the hydrobromination is carried out in a homogeneous liquid medium.

10. Process according to any one of the preceding claims, **characterized in that** the HBr used is in liquid form, liquefied under its saturated vapor pressure or in solution in a solvent.

11. Process according to any one of the preceding claims, **characterized in that** the solvent comprises at least one of the following solvents: benzene, fluorobenzene, chlorobenzene, trifluorotoluene ("BTF"), ethylbenzene, toluene, xylene, cyclohexane, methylcyclohexane, heptane, isooctane, 1,1,1-trichloroethane and dibromomethane, and mixtures thereof.

12. Process according to any one of the preceding claims, **characterized in that** it uses at least one solvent chosen from aliphatic solvents, such as cyclohexane, methylcyclohexane or heptane, and mixtures thereof, optionally as a mixture with at least one other solvent chosen from aromatic solvents such as benzene, toluene or xylene, or halogenated solvents such as monochlorobenzene, and mixtures thereof.

13. Process according to any one of the preceding claims, **characterized in that** the optional solvents arrive in the mixing device at a temperature included in the range of from -50°C to 20°C.

14. Process according to any one of the preceding claims, wherein the compound of formula (I) and the optional solvent arrive in the mixing device **dA** in a stream Fa, while the HBr, the optional solvent and the initiator arrive in the mixing device **dA** in a second stream Fb during step A, the ratio of the streams Fa and Fb being kept substantially constant, and the stream Fr resulting from the mixing of the streams Fa and Fb is directed from the outlet of the mixing device **dA** into the reaction finishing device **dB.**

15. Process according to any one of the preceding claims, wherein the device **dA** comprises at least one reactor and/or at least one mixer, each having:

   - a small total internal volume, generally included in the range of from 1 microliter to 200 liters,
   - a value of the ratio between its internal surface area and its internal volume of greater than 50 $m^2m^{-3}$,
   - a characteristic dimension, namely the shortest distance between two walls facing one another, included in the range of from 1 □m to 1 m.

16. Process according to any one of the preceding claims, **characterized in that** the step of mixing the reagents is carried out under flow rate and pressure conditions such that, within the mixing device **dA,** the circulating reactive fluids have short characteristic times, namely a residence time of less than 10 minutes, preferably included in the range of from 0.01 second to 5 minutes.

17. Process according to any one of the preceding claims, **characterized in that**, during step **A,** the temperature of entry of the compound I into the mixing device **dA** is included in the range of from 0 to 100°C, it being understood that, if the compound I enters alone, it is at a temperature above the melting point Mp of the compound I, whereas if it enters diluted in a solvent, said mixture of compound I and solvent enters dA at a temperature included in the range of from 0 to 50°C.

18. Process according to any one of the preceding claims, **characterized in that**, during step **A,** the HBr entry temperature, when it enters diluted in a solvent, is included in the range of from -50°C to 50°C.

19. Process according to any one of the preceding claims, **characterized in that** the HBr reagent is in an excess of at least 0.1 mol% relative to the reagent of formula (I).

20. Process according to any one of the preceding claims, **characterized in that** the HBr/optional total solvent weight ratio is included in the range of from 1% to 30%.

21. Process according to any one of the preceding claims, **characterized in that** the reagent I/optional total solvent weight ratio is included in the range of from 1% to 80%.

22. Process according to any one of the preceding claims, wherein the radical initiator/HBr ratio is included in the range of from 0.01% to 80%.

23. Process according to any one of the preceding claims, **characterized in that** the hydrobromination is carried out at a temperature included in the range of from -50 to 50°C.

24. Process according to any one of the preceding claims, wherein the hydrobromination reaction is carried out at least partially in adiabatic mode.

25. Process according to any one of the preceding claims, **characterized in that**, at the end of step **A,** the temperature at which the product leaves the device dA is above the crystallization temperature of the product, preferably included in the range of from -50°C to 50°C.

26. Process according to any one of the preceding claims, wherein, at the end of the mixing step **A** and/or at the end of the spontaneous continuation step **B,** a mixture of product, optional solvent and residual HBr is collected, said process also comprising a step **C** of separation and recovery of the product, and the optional recycling of the HBr and/or of the solvent to the mixing device A.

27. Process according to any one of the preceding claims, comprising the production of 11-bromoundecanoic acid from undecylenic acid.

28. Process according to any one of the preceding claims, also comprising a step of ammonolysis so as to form a compound of formula $NH_2\text{-}(CH_2)_n\text{-}R$, such as 11-aminoundecanoic acid.

29. Process according to the preceding claim, also comprising a step of synthesis of polyamide by polymerization of the compound of formula $NH_2\text{-}(CH_2)_n\text{-}R$.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 951932
- CN 1100030 C